# EUROPEAN PATENT APPLICATION

(11) **EP 2 298 392 A1**
(43) Date of publication of application: **23.03.2011**
(21) Application number: 09766555.8
(22) Date of filing: 10.06.2009
(51) Int. Cl.: A61M 5/31, A61M 5/28, A61M 5/315

(54) **INJECTOR**

(30) Priority: 17.06.2008 JP 2008157450
(71) Applicant: Denki Kagaku Kogyo Kabushiki Kaisha, Tokyo 103-8338 (JP); Taisei Kako Co., Ltd., Kita-ku Osaka-shi Osaka 531-0072 (JP)
(72) Inventor: MITSUNO Tohru, Tokyo 103-8338 (JP); MIYATA Yoshiaki, Tokyo 103-8338 (JP); HIOKI Kazutoshi, Itoigawa-shi Niigata 949-0305 (JP); TABATA Kota, Itoigawa-shi Niigata 949-0305 (JP); HOSHIKA Hiromitsu, Itoigawa-shi Niigata 949-0305 (JP); MATSUMOTO Ippei, Ibaraki-shi Osaka 567-0054 (JP); ASAHI Norihiko, Ibaraki-shi Osaka 567-0054 (JP)
(74) Representative: Gulde Hengelhaupt Ziebig & Schneider
(86) International application number: PCT/JP2009/060586
(87) International publication number: WO 2009/154107

(57) **Abstract**

There is provided a injector in which a backstop function is realized by a collar part at a position situated away from a gasket in the direction of the side of the proximal end and adequate sterilization can be carried out, with a plunger rod (in which the collar part is formed) assembled, while aiming to secure airtightness.

(A backstop function is realized by means of a collar part at a position situated away from the gasket in the direction of the side of the proximal end, thereby providing a injector capable of being adequately sterilized with a plunger rod (in which the collar part is formed) assembled, while aiming to secure airtightness.)

A gasket (3) is inserted from a proximal end opening (22) of a barrel (2). Next, a plunger rod (4) is inserted as to establish connection of its distal end and the gasket (3). Then, a finger grip (5) is mounted to a flange (23). A disc part (45) is formed at a position situated away, in the direction of the side of the proximal end, from the gasket, thereby making it possible for the disc part (45) to abut and stop against an aperture edge (521) of the finger grip. There is formed in an outer peripheral edge part of the disc part (45) a communication opening part (451) having a concave shape. At the time of sterilization with hydrogen peroxide gas, the hydrogen peroxide gas which has flowed into an open space (26) from the proximal end opening is permitted to circulate in a semi-hermetically closed space (25) by way of the communication opening part.

## Description

### Technical Field

The present invention is intended for a injector with a backstop function. This invention more particularly relates to a injector which can be adequately sterilized, with a plunger rod inserted into a barrel and a backstopper assembled. The backstop function is a function of preventing the plunger from being pulled out of the barrel of the injector. This backstop function is as follows. There is formed in a plunger rod a collar part. After insertion of the plunger rod into a barrel, a backstopper, which abuts and stops the collar part, is assembled to the proximal end side of the barrel. Accordingly, even when attempting to pull the whole plunger rod out of the barrel, the collar part strikes the backstopper, thereby preventing the plunger rod from being drawn out of the barrel.

### Background Art

Heretofore, there has been proposed, as a treatment for the sterilization of injectors (pre-filled syringes) whose inside is filled in advance with medicinal liquid, a sterilizing treatment which uses hydrogen peroxide gas (see, for example, Patent Literature 1). This sterilizing treatment is carried out in the following steps of a procedure. In the first place, a pre-filled syringe as a target subjected to sterilization is enclosed and hermetically sealed in a specific package body having at a certain portion thereof a section having a permeability to hydrogen peroxide gas. Next, this package body containing the pre-filled syringe is placed in a sterilization tank filled with hydrogen peroxide gas. In this way, the pre-filled syringe is sterilized.

In addition, as a injector with a backstop function, there is also known a injector which is configured such that a stopper is assembled to a barrel whereby, even when attempting to pull a plunger rod off from the barrel, the stopper will abut and stop the gasket at the distal end of the plunger rod, thereby preventing the plunger rod from making a further movement in the pull-off direction (see, for example, Patent Literature 2).

### Citation List

### Patent Literature

Patent Literature 1: JP-A-2006-16053
Patent Literature 2: JP-A-08-294533

### Summary of Invention

### Technical Problem

Incidentally, there has been considered to form, in a plunger rod at a position axially away from the gasket towards the proximal end, a collar part whose outer peripheral surface is almost equal to the inside diameter of a barrel in order that, in addition to the backstop function, the guide of sliding of the plunger rod along in the barrel is ensured more definitely and made smooth and the position where to be backstopped is adjusted for the securing of airtightness. For example, if a collar part is formed at the distal end position of a plunger rod (i.e., at a position adjacent to a gasket), the plunger rod is brought to a stop (backstop) only after the gasket at the distal end of the plunger rod makes a relative movement to a position just next to the proximal end opening of the barrel by manipulation of the plunger rod to the pull side. In this instance, if the plunger rod is twisted causing the plunger rod to tilt relative to the central axis of the barrel, this may result in creation of a gap between the outer peripheral surface of the gasket and the inner wall surface of the barrel, thereby possibly leading to occurrence of a situation incapable of securing airtightness due to the created gap. To sum up, by adjustment in the forming position of the collar part, it becomes possible to not only merely provide a backstop function by the collar part and the backstopper for prevention of "being pulled out", but also to aim to realize other functions.

However, the employing of such a configuration may be considered to give rise to the possibility of not being able to adequately perform a sterilizing treatment. That is, if it is attempted to perform a sterilizing treatment using hydrogen peroxide gas, with a plunger rod inserted in a barrel and a backstopper assembled, the hydrogen peroxide gas will not be spread in every parts. That is to say, since there is formed at the distal end of the plunger rod a collar part, this separates an axial region extending from the gasket at the distal end of the plunger rod to the collar part after insertion of the plunger rod in the barrel, and the axial region defines a semi-hermetically closed space. For this reason, it may be considered that, although hydrogen peroxide gas is allowed to easily enter, in the direction of the inside of the barrel, a region from the proximal end opening of the barrel to the collar part by way of a gap between the proximal end opening of the barrel and the plunger rod cross-sectional surface, the presence of the collar part impedes entry of hydrogen peroxide gas into the semi-hermetically closed space defined ahead of the collar part. As a result, this may give rise to poor sterilization of the inner peripheral surface of the barrel and the outer peripheral surface of the plunger rod, which surfaces exist in the axial region from the gasket to the collar part.

In particular, such inconvenience becomes a critical problem for the case of pre-filled syringes of the type that is sterilized, with the plunger rod inserted and assembled in the barrel. Therefore, solutions thereto have been strongly demanded.

With this situation in mind, the present invention was developed. Accordingly, an object of the present invention is to provide a injector in accordance with which the backstop function is realized by a collar part formed at a position away from the gasket in the direction of the side of the proximal end whereby, even when performing a sterilizing treatment, with the plunger rod (in which the collar part is formed) assembled, it is possible to adequately perform the sterilizing treatment, while aiming to secure airtightness at the point of use.

### Solution to Problem

In order to achieve the aforesaid object, the present invention is intended for a injector comprising a barrel, a gasket which, when inserted liquid tightly and slidably into the barrel from a proximal end opening of the barrel, forms, by partitioning, a medicinal liquid chamber, a plunger rod which, when inserted into the barrel from the proximal end opening of the barrel, is brought into connection, at its distal end, with the gasket, a collar part which is formed so as to project circumferentially from the plunger rod, and a backstopper which is mounted on the side of the proximal end opening of the barrel and which abuts and stops the collar part, thereby preventing the plunger rod from being pulled off. Furthermore, the injector of the present invention has the following characteristic particulars. That is, the collar part is formed so as to project to a position adjacent to the inner peripheral surface of the barrel from the plunger rod at an intermediate position away from the gasket in the direction of the side of the proximal end of the barrel, and has a circulation opening part through which the proximal end side and the distal end side of the barrel are made fluidly communicatable with each other. And the circulation opening part is configured to have an opening size which allows circulation of gas for sterilization from an open space on the side of the proximal end opening of the barrel to a semi-hermetically closed space defined by partitioning between the collar part and the gasket (claim 1).

In the case of this invention, if the plunger rod in the barrel is pulled in the direction of the side of the proximal end opening, this causes the collar part to abut and stop against the backstopper. Therefore, no further pulling operation is allowed and, as a result, the plunger rod is brought to a stop. This prevents occurrence of such a situation that the plunger rod is pulled (dropped) off from the barrel. The stop position of such a plunger rod pulling operation can be changed and adjusted depending on the setting of the intermediate position (i.e., the position of formation of the collar part), thereby making it possible to more definitely aim to secure airtightness. On the other hand, if the injector is placed in an assembled state by inserting a plunger rod into the barrel, this will result in forming, by partitioning, a semi-hermetically closed space between the gasket and the formed collar part. Nonetheless, the circulation opening part is formed in the collar part, and if the assembled injector is placed in an atmosphere filled with gas for sterilization (for example, hydrogen peroxide gas or other like gas), the sterilization gas which has flowed into the open space on the side of the proximal end opening of the barrel is able to adequately circulate also in the semi-hermetically closed space through the circulation opening part, thereby making it possible to adequately sterilize surfaces including the inner peripheral surface of the barrel which defines, by partitioning, the semi-hermetically closed space.

In addition, in accordance with the present invention, the collar part is shaped like a circular disc having a greater diameter than a rod main body of the plunger rod and the circulation opening part is comprised of a concave part formed by concavely removing a portion of the outer peripheral surface of the collar part (claim 2). As a result of such arrangement, the collar part is formed into a circular disc-like shape of greater diameter than the rod main body and therefore the assuredness at the time when the plunger rod slides along in the barrel is enhanced relative to the case of just a rod main body provided with no collar part and it becomes possible for the collar part to function as a slide guide. Moreover, since the circulation opening part is a concave part formed in a portion of the outer peripheral surface of the collar part, this ensures that the outer peripheral edge of the collar part definitely abuts and stops against the backstopper, thereby also making it possible to aim to make the backstop function reliable.

### Advantageous Effects of Invention

As has been described above, in accordance with the injector of either claim 1 or claim 2, the combining of the collar part and the backstopper more definitely secures, in addition to the backstop function, airtightness based on the change and the adjustment in the backstop position. Besides, even if the collar part is formed in order to realize such a function, it is possible, because the circulation opening part is formed, to adequately circulate gas for sterilization by way of the circulation opening part, even when performing a sterilizing treatment by use of gas for sterilization, with the plunger rod inserted and assembled in the barrel. This makes it possible to adequately accomplish sterilization by use of gas for sterilization.

Especially, in accordance with claim 2, the assuredness at the time when the plunger rod is slid along in the barrel is enhanced, thereby making it possible for the collar part to function also as a slide guide. Besides, it becomes possible for the outer peripheral edge of the collar part to more definitely abut and stop against the backstopper, thereby also making it possible to aim to make the backstop function reliable.

### Brief Description of Drawings

Figure 1, comprised of Figure 1(a) and Figure 1(b), shows a injector according to an embodiment of the present invention wherein Figure 1(a) is a side view of the injector and Figure 1(b) is an illustration in which components other than a plunger rod are shown in cross section.
Figure 2 is a perspective view of the injector of Figure 1.
Figure 3 is a perspective view of the plunger rod constituting the injector of Figure 1.
Figure 4 is a cross-sectional illustration taken along line A-A of Figure 1(b).
Figure 5 shows a corresponding view to Figure 1(b) when the plunger rod is pulled, in the pull direction, to a limit position, and a partially enlarged view.
Figure 6, comprised of Figure 6(a) and Figure 6(b), shows a finger grip wherein Figure 6(a) is a perspective view of the finger grip as viewed from the proximal end side and Figure 6(b) is a perspective view of the finger grip as viewed from the distal end side.
Figure 7(a) is a corresponding view to Figure 4, and shows another embodiment of a circulation opening part which is formed in a disc part, and Figure 7(b) is a corresponding view to Figure 4, and shows still another embodiment other than that shown in Figure 7(a).
Figure 8 is a corresponding view to Figure 4, and shows another embodiment of the circulation opening part other than that shown in Figure 7.

### Description of Embodiments

Hereinafter, embodiments of the present invention will be described with reference to the drawings.

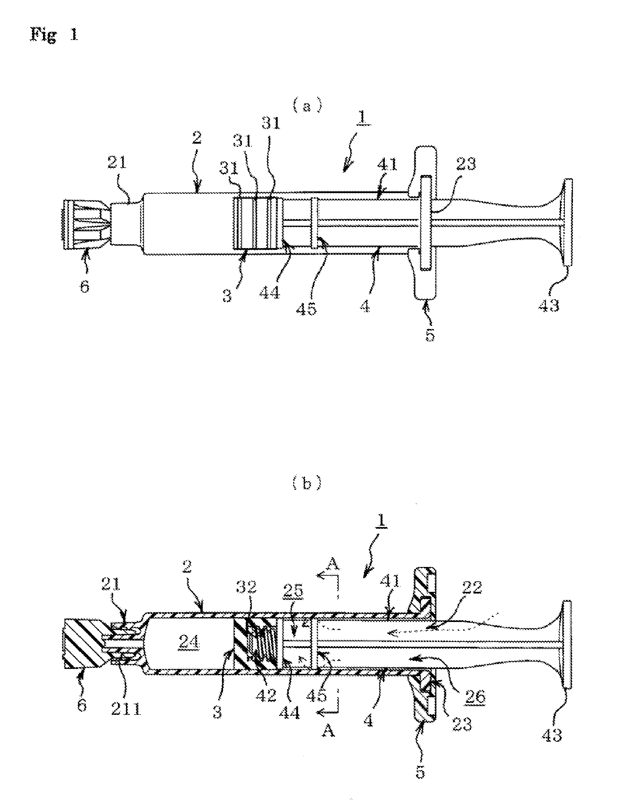

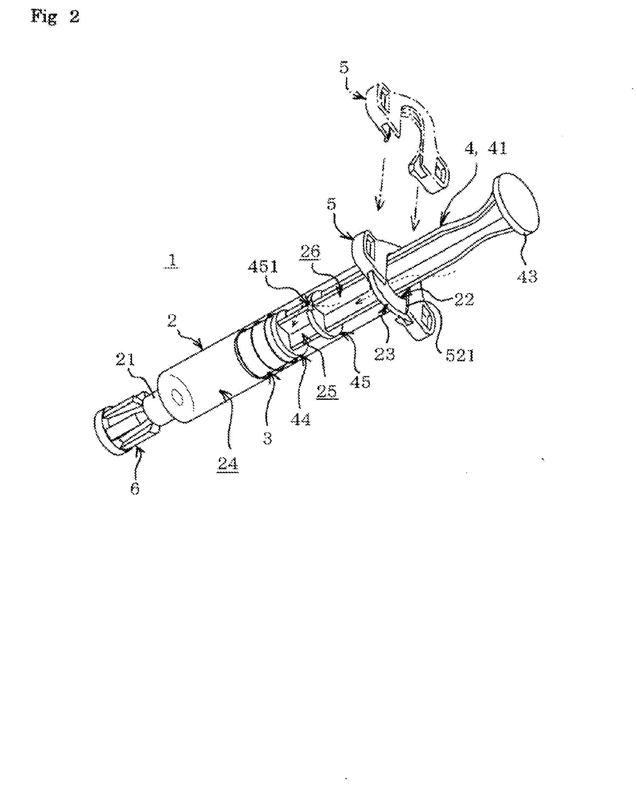

Referring to Figure 1 and Figure 2, there is shown a injector 1 according to an embodiment of the present invention. In the figures, reference numeral 2 denotes a barrel which constitutes a syringe barrel of the injector 1; reference numeral 3 denotes a gasket; reference numeral 4 denotes a plunger rod; reference numeral 5 denotes a finger grip; and reference numeral 6 denotes a top cap for sealing.

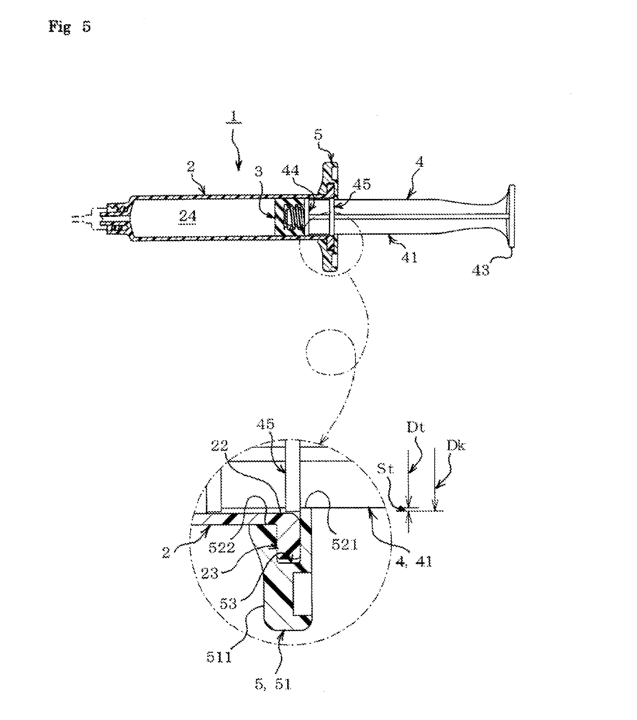

A nozzle 21 is formed at the distal end of the barrel 2. The top cap 6 is detachably attached to the nozzle 21. The nozzle 21 is configured in a double tube structure (see Figure 1(b)). The top cap 6 is fitted onto an inner tube part 211 in fluid communication with the inside of the barrel 2, thereby liquid tightly sealing a distal end opening of the inner tube part 211. In addition, there are formed on the side of the proximal end of the barrel 2 a proximal end opening 22 and a flange 23 projecting on the outer peripheral side. The finger grip 5 is attached to the flange 23. In the following description, what is meant by the term "distal end side" is the left-hand side in Figures 1 and 5 while, on the other hand, what is meant by the term "proximal end side" is the right-hand side in Figures 1 and 5.

The gasket 3 is closely fitted, through the proximal end opening 22 of the barrel 2, into the barrel 2, and is configured to be slid along in the barrel 2 by pushing or pulling of a plunger rod 4 to be hereinafter described. A plurality of ribs 31, 31, ... having a slightly expanded diameter are integrally formed on the outer peripheral surface of the gasket 3. Each rib 31 is in close contact, in a liquid tight manner, with the inner peripheral surface of the barrel 2, thereby blocking leakage of medicinal liquid to be held in the barrel 2 on the distal end side relative to the gasket 3. In addition, there is formed in the gasket 3 a concave part 32 which opens on the proximal end side of the barrel 2 and which has in its inner peripheral surface a thread groove, and a thread part 42 (to be hereinafter described) of the plunger rod 4 is screwed in the concave part 32 so that the gasket 3 is integrally coupled to the distal end of the plunger rod 4. With the plunger rod 4 and the gasket 3 made integral with each other, there is constituted a plunger. And, a medicinal liquid chamber 24 for accommodating medicinal liquid is defined, by partitioning, in the inside of the barrel 2 ahead of the gasket 3.

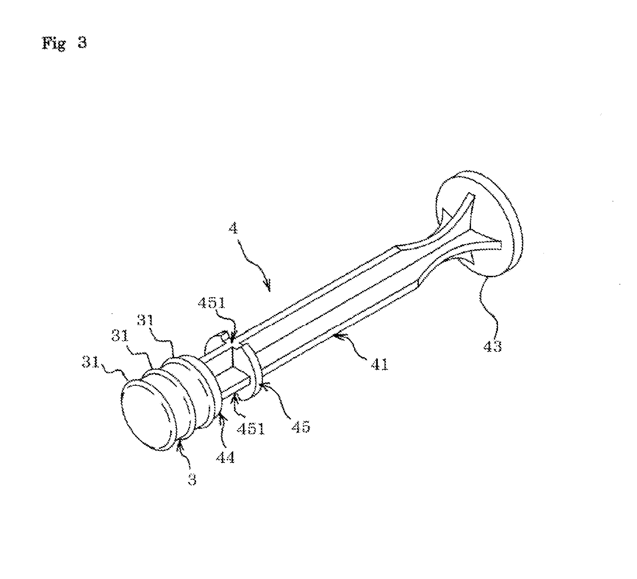

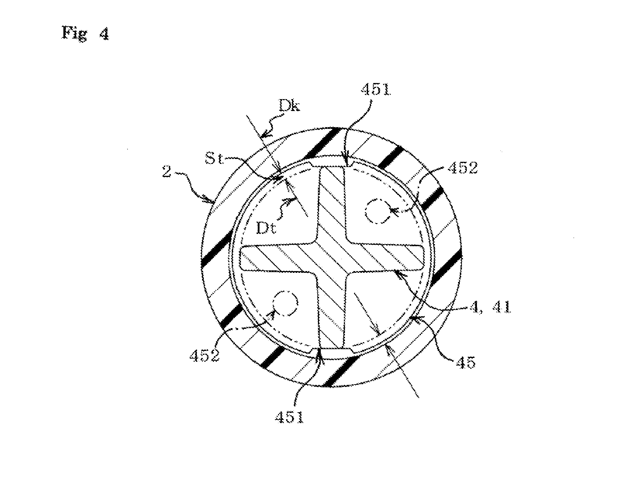

As also shown in Figure 3, the plunger rod 4 includes a rod main body 41 which is shaped like a cross in transverse cross section (see, in addition, Figure 4), a thread part 42 which projects from the distal end position of the rod main body 41 in the direction of the distal end of the barrel 2 and an operation part 43 which extends circumferentially from the proximal end position of the rod main body 41. In addition to these parts, the plunger rod 4 further includes a disc part 44 and a disc part 45 at the distal end position of the rod main body 41 and at the intermediate position of the rod main body 41 at a given distance away from the distal end position in the direction of the proximal end side, respectively. The disc part 44 at the distal end position has an outside diameter capable of abutting and covering almost the entire backside of the gasket 3. And by connecting the gasket 3 to the thread part 42, the gasket 3 is borne from the backside thereof for homogeneous transmission of a pushing force from the plunger rod 4 to the gasket 3. On the other hand, the disc part 45 has an outside diameter *Dk* which is set as follows (see Figure 4). That is, the outside diameter *Dk* is smaller, just by such a slight extent that it becomes minimum in the range that allows both the disc parts 44, 45 to advancingly and retreatingly move within the barrel 2, than the inside diameter of the barrel 2. But the outside diameter *Dk* is larger, by a corresponding dimension to an abutment-stop margin *St* (to be hereinafter described), than the diameter, *Dt,* of a virtual circular arc (i.e., a circular arc represented in Figure 4 by chain double-dashed line) formed by connecting together outer peripheral end edges of the cross-shaped body constituting the rod main body 41, and the diameter *Dt* is hereinafter referred to as the "reference diameter". The disc part 45, whose diameter is rendered larger just by an amount corresponding to the abutment-stop margin *St*, constitutes a collar part for preventing the plunger rod 4 from being pulled off from the barrel 2. In other words, when the plunger rod 4 is pulled in the direction of the proximal end side thereof (i.e., the pull-off side), as shown in Figure 5, the disc part 45 abuts and stops against the finger grip 5, thereby preventing the plunger rod 4 from making a further movement in the direction of the pull-off side. The finger grip 5 constitutes a backstopper, and a back-stop function based on the combination of the finger grip 5 and the collar part 45 will be described hereinafter.

And, communication opening parts 451, 451 serving as a circulation opening part are formed in the disc part 45 by concavely cutting away some parts of the outer peripheral edge thereof into concave parts, and a predetermined gas for sterilization is circulated, through these communication opening parts 451, 451, between the proximal end side and the distal end side in the barrel 2. That is, fluid communication between a semi-hermetically closed space 25 sandwiched between both the disc parts 44, 45 in the barrel 2 and an open space 26 in the barrel 2 which is a space situated, relative to the disc part 45, on the side of proximal end and which is brought into fluid communication through the proximal end opening 22 with the outside, is established to such an extent that makes the gas for sterilization (for example, hydrogen peroxide gas) circulatable through the communication opening parts 451, 451. What is meant here by the phrase "such an extent that makes the gas for sterilization circulatable" is that the communication opening part 451 is an opening having such a degree of size that the opening area thereof falls, for example, either in the range between 1.0 and 4.0 mm² or in the range between 1.0 and 5.0 mm², but does not include an opening such as a very small gap that is defined between the outer peripheral surface of the disc part 45 in the absence of the communication opening parts 451, 451 and the inner peripheral surface of the barrel 2, and incapable of adequate circulation of gas for sterilization therethrough. There is no upper limit to the aforesaid opening area, and although, with respect to the opening area, larger is better for the reason that gas for sterilization will become more circulatable, it is advisable to set the shape and the layout of the communication opening part 451 such that the opening area becomes maximum in the range within which the backstop function can be ensured, as will be described also in the section of other embodiments of the present invention to be hereinafter explained. In this sense, it suffices that the communication opening part 451 is an opening having, as the level that makes the gas for sterilization circulatable, a degree of size falling in the range, for example, between 1.0 and 7.0 mm².

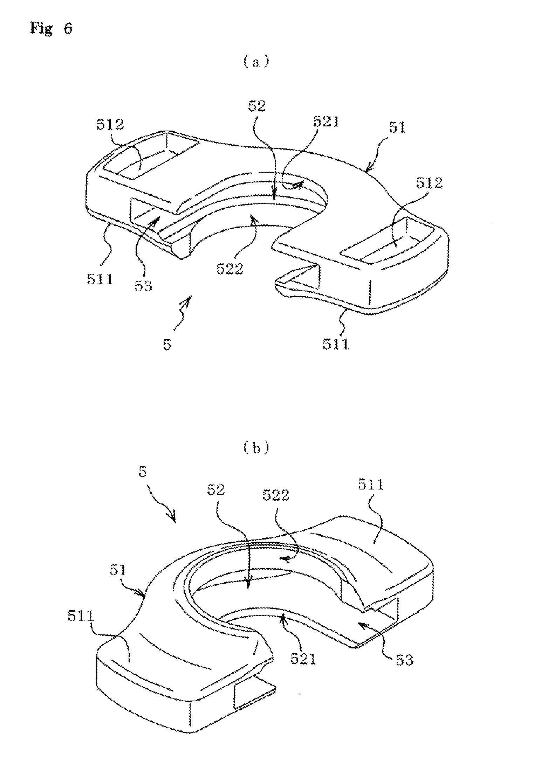

Next, the finger grip 5 is provided by forming a portion which more widely stretches out from the flange 23 of the barrel 2 to the right- and left-hand sides so as to form a finger hook portion grasped when performing a injection operation with the injector 1. The attaching of the finger grip 5 to the flange 23 is carried out after insertion of the plunger rod 4 into the barrel 2, the reason for which is that the finger grip 5 serves also as a backstopper for preventing the plunger rod 4 from being pulled off. Referring to Figure 6, a description will be made with regard to details of the configuration of the finger grip 5. The finger grip 5 includes: a grip main body 51 which has grip parts 511, 511 projecting bilaterally across the flange 23 of the barrel 2 and which has a thickness capable of containing the amount of thickness of the flange 23; an aperture part 52 into which the barrel 2 is inserted passing completely through the central position of the grip main body 51 (penetration in the vertical direction of Figure 6); and a concave groove part 53 which is able to accommodate the flange 23, being in fluid communication with the aperture part 52. In addition, an aperture edge 521, which is the aperture edge of the aperture part 52 and is situated on one side (the proximal end side of either Figure 1 or Figure 5) across the opening of the concave groove 53, is configured such that it has the reference diameter *Dt* (see Figure 4), whereas an aperture edge 522 situated on the other side (the distal end side of either Figure 1 or Figure 5) is configured such that it has a diameter approximately equal to the outside diameter of the barrel 2. In addition, reference numeral 512 in Figure 6 denotes a concave part which is provided for the reduction in weight of the grip main body 51 and for the prevention of sink after formation.

And, as shown in Figure 2, the finger grip 5 is attached by being laterally engaged into the flange 23 of the barrel 2. More specifically, the aperture edge 522 on the distal end side is fitted onto the outer peripheral surface of the barrel 2 adjacent to the flange 23, and the finger grip 5 is laterally engaged so that the flange 23 enters the inside of the concave groove 53 whereby the finger grip 5 is coupled to the flange 23 and becomes immobile as shown in solid line of Figure 2. In this state, the proximal end opening 22 of the barrel 2 is narrowed down to the reference diameter *Dt* by the aperture edge 521 on the proximal end side, as shown in Figure 5. Consequently, when the plunger rod 4 is pulled in the direction of the proximal end side, the disc part 45 abuts and stops against the aperture edge 521 just for the abutment-stop margin *St* because the disc part 45 of greater diameter has the outside diameter *Dk* larger by the abutment-stop margin *St* than the reference diameter *Dt*, thereby preventing the plunger rod 4 from making a further pull-off movement in the direction of the proximal end side. This realizes a backstop function.

In the way as described above, it becomes possible to definitely prevent, owing to abutment-stop against the disc part 45, the plunger rod 4 from being pulled off. In addition, as shown in Figure 5, it also becomes possible that the upper limit when expanding the medicinal liquid chamber 24 by pulling of the plunger rod 4, i.e., the limit of movement of the plunger rod 4 in the direction of the pull side), can be adjusted, corresponding to the layout position of the disc part 45. By such adjustment, the function of coaxially holding the plunger rod 4 and the barrel 2 is further ensured, thereby making it possible to not only more definitely aim to secure airtightness, but also in addition thereto, to control the greatest suctionable amount of medicinal liquid. In addition, the plunger rod 4, when being moved, is slidingly guided against the internal peripheral surface of the barrel 2 by two parts (i.e., the gasket 3 and the disc part 45) at a distance from each other, thereby making it possible for the plunger rod 4 to more definitely and smoothly perform its sliding operation.

Next, a description will be made with regard to the flow of process up to the shipping of the foregoing injector as a pre-filled syringe. The flow of process up to the shipping includes an individual sterilization step, a medicinal liquid filling/assembly step, a packaging step and a final sterilization step which are performed in that order. The individual sterilization step is a step in which component parts, such as the barrel 2, the gasket 3, the plunger rod 4, the finger grip 5 and the top cap 6, are individually subjected to a sterilizing treatment such as, for example, a high-temperature steam sterilizing treatment. In the medicinal liquid filling/assembly step, with the top cap 6 and the gasket 3 assembled to the barrel 2, the medicinal liquid chamber 24 is filled with a medicinal liquid by means of, for example, a vacuum filling method. Thereafter, the plunger rod 4 is inserted so that its distal end is coupled to the gasket 3. Then, the finger grip 5 is mounted to the flange 23 to complete a pre-filled syringe (see a state shown in Figure 1). In the packaging step, the pre-filled syringe after medicinal liquid filling is placed in a predetermined package body which is then hermetically sealed. With the syringe packed in the package body, the final sterilization step is carried out using hydrogen peroxide gas. The package body is in the form of a blister container or a package bag, and one that has at a certain part thereof a permeative part which allows transmission of hydrogen peroxide gas therethrough can be used. And, in the final sterilization step, the pre-filled syringe in a state of being accommodated in the package body is placed in a sterilization tank filled with hydrogen peroxide gas for a predetermined length of time to perform sterilization by contact with hydrogen peroxide gas.

In the final sterilization step, hydrogen peroxide gas, after having passed through the package body, enters the open space 26 from the proximal end opening 22 of the barrel 2, as shown by dotted line in either Figure 1(b) or Figure 2. After that, the hydrogen peroxide gas enters, by way of the communication opening parts 451, 451 of the disc part 45 (see, in addition, also Figure 4), the semi-hermetically closed space 25. Accordingly, in spite of the fact that the provision of the disc part 45 results in forming, by partitioning, the semi-hermetically closed space 25 between the gasket 3 and the disc part 45, the circulating of hydrogen peroxide gas through the communication opening parts 451, 451 is ensured, thereby making it possible to adequately sterilize surfaces in the semi-hermetically closed space 25 (more specifically, the inner peripheral surface of the barrel 2, the outer peripheral surface of the plunger rod 4 and so on).

### Other Embodiments

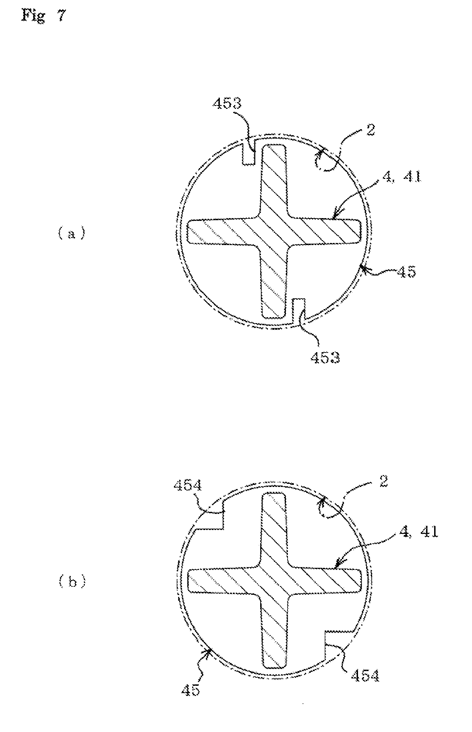

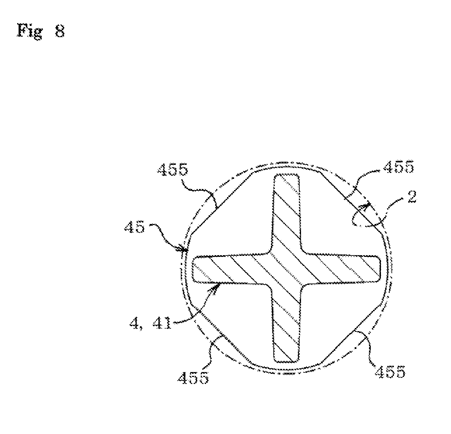

Also note that the present invention is not limited to the foregoing embodiment and therefore includes other various embodiments. That is, although in the foregoing embodiment the groove-shaped communication opening parts 451, 451 (see Figure 4) are shown as a communication opening part to be formed in the disc part 45, it is possible to select other communication opening parts different in shape, layout and numeric quantity if they fall within the range capable of adequately ensuring the circulation of gas for sterilization and, in addition, capable of ensuring that the disc part 45 and the aperture edge 521 of the finger grip 5 are placed in a mutual abutment-stop state. For example, it may be possible to employ, as a circulation opening part for ensuring the circulation of gas for sterilization, a through hole represented by reference numeral 452 in Figure 4, a communication opening part in the shape of a slit groove represented by reference numeral 453 in Figure 7(a), a cutaway opening part represented by reference numeral 454 in Figure 7(b), or a cut part represented by reference numeral 455 in Figure 8.

In the foregoing embodiment, hydrogen peroxide gas is used as a gas for sterilization, which, however, should not be considered restrictive in any way. For example, other types of sterilization gases such as ethylene oxide gas may be employed.

In addition, there is shown in the foregoing embodiment a case in which the present invention is applied to a treatment for the sterilization of a pre-filled syringe, which, however, should not be considered restrictive in any way. The present invention can be applied to a case in which a sterilization treatment is carried out using a gas for sterilization, in an assembly state in which the plunger rod 4 is inserted in the barrel 2.

Furthermore, there is shown as a backstopper in the foregoing embodiment one that is annexed to the finger grip 5, which, however, should not be considered restrictive in any way. The backstopper may be in any other form as long as it has a portion (for example, a portion corresponding to the aperture edge 521 of the finger grip 5) capable of abutment-stop against the peripheral part of a portion corresponding to the abutment-stop margin of the disc part 45 which serves as a collar part. Moreover, the present invention can be applied to whatever backstopper that is able to realize a backstop function by abutment-stop against the disc part 45 other than the aforesaid peripheral edge part. In addition, there is shown the rod main body 41 of the plunger rod 4 which is shaped like a cross in cross section, which, however, should not be considered restrictive. For example, the rod main body 41 may be shaped like a letter "H" or a square in cross section.

### Example

The final sterilization step was performed on both embodying and comparative examples. Thereafter, testing for comparatively verifying, with regard to the effect of sterilization, an embodying example of the present invention and a comparative example, was conducted. As the final sterilization step, the following was carried out. That is, a respective one of the pre-filled syringes in a state of being accommodated in a package body was placed in a sterilization tank filled with hydrogen peroxide gas and then sterilization by contact with hydrogen peroxide gas was carried out. The embodying example is a pre-filled syringe when configured to use the plunger rod 4 in which the communication opening part 451 is formed, as described in the foregoing embodiment. The comparative example is a pre-filled syringe when configured to use a plunger rod in which the communication opening part 451 is not formed. Here, the opening area of the communication opening part 451 of the embodying example is 1.1 mm².

In the sterilization treatment in the final sterilization step, the following packaging manner was employed for both the embodying example and the comparative example. In other words, each pre-filled syringe after assembly was pillow packaged using such a sheet of polyethylene (lateral width: 140 mm; length: 185 mm) that a 14 mm-portion of the lateral width is replaced with covering by TYVEK (Trademark, DuPont) extending over the entire length in the longitudinal direction, thereby placing the pre-filled syringe in a state of being enclosed in a 70 by 185-mm packaging body.

The conditions of sterilization in the final sterilization step in which to place the embodying example and the comparative example in a sterilization tank, were set as follows. That is, a 35% hydrogen peroxide solution was dropped into a sterilization box (sterilization tank) whose inner volume is 0.600 m³ for a dropping time of 90 minutes at a dropping volume of 5.5 g/min. In order to place the sterilization box in a hot-dry state at the time of sterilization, conditioning was made to control the initial inside temperature and the initial inside humidity at 35 degrees Centigrade and at 5R% or lower, respectively.

The results of the above testing shows that the embodying example provided with the communication opening part 451 achieved a sterility assurance level 10⁻⁶ (SAL10-6) while on the other hand the comparative example not provided with a communication opening part failed to provide sterilization to a sterility assurance level 10⁻⁶. This confirms the fact that, by forming a communication opening part having an opening area of 1.1 mm², it becomes possible to accomplish a predetermined sterilization effect.

### Industrial Applicability

The present invention provides a injector which is distributed and provided as a pre-filled syringe in the filed of medicine or other like field.

### Reference Signs List

- 1: injector
- 2: barrel
- 3: gasket
- 4: plunger rod
- 5: finger grip (backstopper)
- 22: proximal end opening
- 24: medicinal liquid chamber
- 25: semi-hermetically closed space
- 26: open space
- 45: disc part (collar part)
- 451, 453: communication opening part (circulation opening part, concave part)
- 452: through hole (circulation opening part)
- 454: cutaway opening part (circulation opening part)
- 455: cut part (circulation opening part)

## Claims

1. A injector which comprises:
a barrel;
a gasket which, when inserted liquid tightly and slidably into said barrel from a proximal end opening of said barrel, defines, by partitioning, a medicinal liquid chamber;
a plunger rod which, when inserted into said barrel from said proximal end opening of said barrel, is brought into connection, at its distal end, with said gasket;
a collar part which is formed so as to project circumferentially from said plunger rod; and
a backstopper which is mounted on the side of said proximal end opening of said barrel and
which abuts and stops said collar part, thereby preventing said plunger rod from being pulled off;
wherein said collar part is formed so as to project to a position adjacent to the inner peripheral surface of said barrel from said plunger rod at an intermediate position away from said gasket in the direction of the side of the proximal end of said barrel, and has a circulation opening part through which the proximal end side and the distal end side of said barrel are made fluidly communicatable with each other; and
wherein said circulation opening part is configured to have an opening size which allows circulation of gas for sterilization from an open space on the side of said proximal end opening of said barrel to a semi-hermetically closed space defined by partitioning between said collar part and said gasket.

2. The injector as set forth in claim 1,
wherein said collar part is shaped like a circular disc having a greater diameter than a rod main body of said plunger rod; and
wherein said circulation opening part is comprised of a concave part formed by concavely removing a portion of the outer peripheral surface of said collar part.
